# EUROPÄISCHE PATENTSCHRIFT

(11) **EP 1 280 776 B1**
(45) Veröffentlichungstag und Bekanntmachung des Hinweises auf die Patenterteilung: **21.10.2009**
(21) Anmeldenummer: 01925566.0
(22) Anmeldetag: 24.04.2001
(51) Int. Cl.: C07D 213/56, A61K 31/44, A61P 19/02, A61P 7/02, A61P 27/06, A61P 35/00, C07D 401/12, A61K 31/16, C07C 235/42, C07D 217/22

(54) **SUBSTITUIERTE BENZOESÄUREAMIDE UND DEREN VERWENDUNG ZUR HEMMUNG VON ANGIOGENESE**
SUBSTITUTED BENZOIC ACID AMIDES AND USE THEREOF FOR THE INHIBITION OF ANGIOGENESIS
AMIDES D'ACIDE BENZOIQUE SUBSTITUES ET UTILISATION DESDITS AMIDES POUR INHIBER L'ANGIOGENESE

(30) Priorität: 25.04.2000 DE 10021246
(43) Veröffentlichungstag der Anmeldung: 05.02.2003
(73) Patentinhaber: Bayer Schering Pharma Aktiengesellschaft, 13353 Berlin (DE)
(72) Erfinder: HUTH, Andreas, 12437 Berlin (DE); SEIDELMANN, Dieter, 12159 Berlin (DE); THIERAUCH, Karl-Heinz, 14169 Berlin (DE)
(86) Internationale Anmeldenummer: PCT/EP2001/004627
(87) Internationale Veröffentlichungsnummer: WO 2001/081311

(56) Entgegenhaltungen:
- WO-A-98/23581
- FR-A- 1 600 541
- KANG S -K ET AL: "Copper-Catalyzed Coupling of Polymer Bound Iodide with Organostannanes" TETRAHEDRON LETTERS,NL,ELSEVIER SCIENCE PUBLISHERS, AMSTERDAM, Bd. 39, Nr. 19, 7. Mai 1998 (1998-05-07), Seiten 3011-3012, XP004115741 ISSN: 0040-4039
- SROGL, JIRI ET AL: "Bio- organometallic Organosulfur Chemistry. Transition Metal-Catalyzed Cross-Coupling Using Coenzyme M or Thioglycoli Acid as the Leaving Group" J. AM. CHEM. SOC. (1999), 121(40), 9449-9450 , 1999, XP002172290
- SHI, CHONGSHENG ET AL: "Biradicals from thermolysis of N-[2-(1-alkynyl)phenyl]-N'- phenylcarbodiimides and their subsequent transformations to 6H-indolo[2,3-b]quinolines" J. ORG. CHEM. (1999), 64(3), 925 - 932 , 1999, XP002172291
- OHNO, MASATOMI ET AL: "PhI(OAc)2-Promoted Rearrangement of the Hydroxyl Group: Ring Expansion of 4-Hydroxy-2-cyclobutenone to 2(5H)-Furanone in Comparison with Ring Cleavage of the.alpha.-Hydroxycycloalkanone to the.omega.-Formyl Ester" J. ORG. CHEM. (1999), 64(25), 8995 - 9000 , 1999, XP002172292
- DATABASE BEILSTEIN [Online] BEILSTEIN INSTITUTE FOR ORGANIC CHEMISTRY, FRANKFURT/MAIN, DE; 2-pheneethyl-benzoic acid methyl ester, Database accession no. 3310153 XP002172293 & JUSTUS LIEBIGS ANN. CHEM., 1928, Seite 267
- DATABASE BEILSTEIN [Online] BEILSTEIN INSTITUTE FOR ORGANIC CHEMISTRY, FRANKFURT/MAIN, DE; 2-styryl-benzoic acid methyl ester, Database accession no. 3202131 XP002172294 & CAN J. CHEM, Bd. 73, Nr. 10, 1995, Seiten 1660-1665,
- DATABASE BEILSTEIN [Online] BEILSTEIN INSTITUTE FOR ORGANIC CHEMISTRY, FRANKFURT/MAIN, DE; 3-benzyl-benzoic acid methyl ester, Database accession no. 2452550 XP002172295 & MAGN. RESON. CHEM., Bd. 27, Nr. 6, 1989, Seiten 585-591,

## Beschreibung

Die Erfindung betrifft substituierte Benzoesäureamide und deren Verwendung als Arzneimittel zur Behandlung von Erkrankungen, die durch persistente Angiogenese ausgelöst werden sowie deren Zwischenprodukte zur Herstellung der Benzoesäureamide.

Persistente Angiogenese kann die Ursache für verschiedene Erkrankungen wie Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen und Artheriosklerose sein oder zu einer Verschlimmerung dieser Erkrankungen führen.

Eine direkte oder indirekte Inhibition des VEGF-Rezeptors (VEGF = vaskulärer endothelialer Wachstumsfaktor) kann zur Behandlung derartiger Erkrankungen und anderer VEGF-induzierter pathologischer Angiogenese und vaskularer permeabiler Bedingungen, wie Tumor-Vaskularisierung, verwendet werden. Beispielsweise ist bekannt, daß durch lösliche Rezeptoren und Antikörper gegen VEGF das Wachstum von Tumoren gehemmt werden kann.

Die persistente Angiogenese wird durch den Faktor VEGF über seinen Rezeptor induziert. Damit VEGF diese Wirkung entfalten kann ist es nötig, daß VEGF am Rezeptor bindet und eine Tyrosinphosphorylierung hervorgerufen wird.
Es ist sind nur entfernte Derivate der hier beanspruchten Verbindungen als Calpaininhibitoren (WO 9823581, WO 9825883) Phospholipase A2-Inhibitoren (WO 9700583) Prostaglandin D2 Antagonisten (WO 9700853), Neurokinin A Antagonisten (WO 9516682), Tranquilizer (US 3892752) oder Anorexigenics (FR 1600541) beschrieben worden.

Eine Wirkung dieser bekannten Verbindungen im Zusammenhang mit VEGF wurde bisher nicht beschrieben.

Als allgemeiner Stand der Technik der derzeitigen Erfindung können die folgenden Veröffentlichungen zitiert werden :

In der PCT Anmeldung WO 98/23581, werden Benzamidoaldehyde Derivate und deren Anwendung als Inhibitoren von Cystein-Proteasen beschrieben.

In dem französischen Patent FR 1,600,541, werden Verfahren zur Herstellung von neuen alpha-substiuierten 2-Aminomethylbenzylalkoholen beschrieben.

Es wurde nun gefunden, daß Verbindungen der allgemeinen Formel I 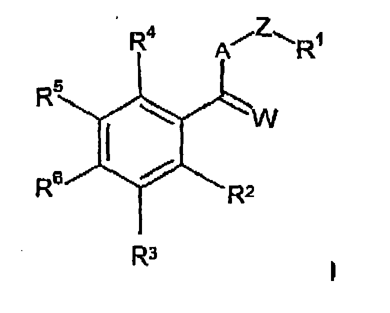 in der
- A: für die Gruppe =NR⁷ steht,
- W: für Sauerstoff, zwei Wasserstoffatome oder die Gruppe =NR⁸ steht,
- Z: für eine Bindung, die Gruppe =NR¹⁰, verzweigtes oder unverzweigtes C₁₋₁₂-Alkyl steht,
- R¹: für gegebenenfalls ein- oder mehrfach mit Halogen oder C₁₋₆-Alkyl substituiertes verzweigtes oder unverzweigtes C₁₋₆-Alkyl, C₂₋₁₂-Alkenyl oder C₃₋₁₂-Alkinyl oder gegebenenfalls ein- oder mehrfach mit Halogen oder C₁₋₆-Alkyl substituiertes C₃₋₁₀-Cycloalkyl oder C₃₋₁₀-Cycloalkenyl oder unsubstituiertes oder gegebenenfalls ein- oder mehrfach mit Halogen, C₁₋₆-Alkyl, C₁₋₆-Alkoxy, ein- oder mehrfach mit Halogen substituiertes C₁₋₆-Alkyl oder C₁₋₆-Alkoxy substituiertes Aryl oder Heteroaryl steht,
- R² und R³: für Wasserstoff, eine OH-Gruppe oder die Gruppe XR¹¹ stehen,
- X: für C₂₋₆-Alkyl, C₂₋₆-Alkenyl oder C₂₋₆-Alkinyl steht,
- R¹¹: unsubstituiertes oder gegebenenfalls ein- oder mehrfach mit Halogen, C₁₋₆-Alkyl, C₁₋₆-Alkoxy oder Hydroxy, substituiertes Phenyl, Pyrimidinyl oder Pyridyl bedeutet,
- R⁴ ,R⁵ und R⁶: für Wasserstoff, stehen,

- R⁷: für Wasserstoff steht,
- R⁸ und R¹⁰: für Wasserstoff oder C₁₋₆-Alkyl stehen, wobei R² und
R³ nicht gleichzeitig für Wasserstoff stehen und falls R² für eine OH - Gruppe steht, R³ nicht für Wasserstoff steht und falls R³ für eine OH-Gruppe steht, R² nicht für Wasserstoff steht sowie deren Isomeren und Salze, Angiogenese stoppen und damit das Wachstum und ein Ausbreiten von Tumoren verhindern.

Unter Alkyl ist jeweils ein geradkettiger oder verzweigter Alkylrest, wie beispielsweise Methyl, Ethyl, Propyl, Isopropyl, Butyl, Isobutyl, sek. Butyl, Pentyl, Isopentyl oder Hexyl zu verstehen, wobei C₁₋₄-Alkylreste bevorzugt werden.

Unter Cycloalkyl ist jeweils Cyclopropyl, Cyclobutyl, Cyclopentyl, Cyclohexyl oder Cycloheptyl, Cyclooctyl, Cyclononyl oder Cyclodecyl zu verstehen.

Unter Cycloalkenyl ist jeweils Cyclobutenyl, Cyclopentenyl, Cyclohexenyl, Cycloheptenyl, Cyclooctenyl, Cyclononenyl oder Cyclodecenyl zu verstehen, wobei die Anknüpfung sowohl an der Doppelbindung wie auch an den Einfachbindungen erfolgen kann.

Unter Halogen ist jeweils Fluor, Chlor, Brom oder Jod zu verstehen.

Die Alkenyl- und Alkinyl-Substituenten sind jeweils geradkettig oder verzweigt und enthalten 2 - 6, bevorzugt 2 - 4 C-Atome. Beispielsweise seien die folgenden Reste genannt: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, Ethinyl, Prop-1-in-1-yl, But-1-in-1-yl, But-2-in-1-yl, But-3-en-1-yl, Allyl.

Der Arylrest hat jeweils 6 -12 Kohlenstoffatome wie beispielsweise Naphthyl, Biphenyl und insbesondere Phenyl.

Der Heteroarylrest kann jeweils benzokondensiert sein. Beispielsweise seien als 5-Ringheteroaromaten genannt: Thiophen, Furan, Oxazol, Thiazol, lmidazol und Benzoderivate davon und als 6-Ring-Heteroaromaten Pyridin, Pyrimidin, Triazin, Chinolin, Isochinolin und Benzoderivate.

Der Aryl- und der Heteroarylrest kann jeweils 1-, 2- oder 3-fach gleich oder verschieden substitutiert sein mit Halogen, C₁₋₄-Alkoxy, Nitro, Trifluormethyl, Trifluormethoxy, Cyano, SO_{q}R⁵ oder C₁₋₄-Alkyl, wobei q für 0 - 2 steht.

Ist eine saure Funktion enthalten sind als Salze die physiologisch verträglichen Salze organischer und anorganischer Basen geeignet wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Ainino-2,3,4-butantriol.

Ist eine basische Funktion enthalten sind die physiologisch verträglichen Salze organischer und anorganischer Säuren geeignet wie Salzsäure, Schwefelsäure, Phosphorsäure, Zitronensäure, Weinsäure, Fumarsäue u.a.

Als besonders wirksam haben sich solche Verbindungen der allgemeinen Formel I erwiesen, in der
- A: für die Gruppe =NR⁷ steht,
- W: für Sauerstoff, Schwefel oder zwei Wasserstoffatome steht,
- Z: für eine Bindung, die Gruppe =NR¹⁰ oder verzweigtes oder unverzweigtes C₁₋₁₂-Alkyl steht,
- R¹: für gegebenenfalls ein- oder mehrfach mit Halogen oder C₁₋₆-Alkyl substituiertes verzweigtes oder unverzweigtes C₁₋₆-Alkyl oder gegebenenfalls ein- oder mehrfach mit Halogen oder C₁₋₆-Alkyl substituiertes C₃₋₁₀-Cycloalkyl oder unsubstituiertes oder gegebenenfalls ein- oder mehrfach mit Halogen, C₁₋₆-Alkyl, C₁₋₆-Alkoxy, ein- oder mehrfach mit Halogen substituiertes C₁₋₆-Alkyl oder C₁₋₆-Alkoxy substituiertes Phenyl, Pyridyl, Naphthyl, Chinolyl, Isochinolyl, Indanyl, Tetralinyl, Indolyl, Thienyl, Indazolyl oder Benzothiazolyl steht,
- R² und R³: für Wasserstoff, eine OH-Gruppe oder die Gruppe XR¹¹ stehen,
- X: für C₂₋₆-Alkyl, C₂₋₆-Alkenyl oder C₂₋₆-Alkinyl steht,
- R¹¹: unsubstituiertes oder gegebenenfalls ein- oder mehrfach mit Halogen, C₁₋₆-Alkoxy oder Hydroxy, substituiertes Phenyl, Pyrimidinyl oder Pyridyl bedeutet,
- R⁴, R⁵, R⁶ und R⁷: für Wasserstoff stehen,
- R⁸ und R¹⁰: für Wasserstoff oder C₁₋₆-Alkyl stehen, wobei R² und
R³ nicht gleichzeitig für Wasserstoff stehen und falls R² für eine OH - Gruppe steht, R³ nicht für Wasserstoff steht und falls R³ für eine OH-Gruppe steht, R² nicht für Wasserstoff steht, bedeuten, sowie deren Isomeren und Salze.

Als ganz besonders wirksam haben sich solche Verbindungen der allgemeinen Formel I verwiesen, in der
- A: für die Gruppe =NR⁷ steht,
- W: für Sauerstoff oder ein oder zwei Wasserstoffatome steht,
- Z: für eine Bindung, die Gruppe =NR¹⁰ oder verzweigtes oder unverzweigtes C₁₋₁₂-Alkyl steht,
- R¹: für verzweigtes oder unverzweigtes C₁₋₆-Alkyl, oder gegebenenfalls ein- oder mehrfach mit Halogen oder C₁₋₆-Alkyl substituiertes C₃₋₁₀-Cycloalkyl oder unsubstituiertes oder gegebenenfalls ein- oder mehrfach mit Halogen, C₁₋₆-Alkyl, C₁₋₆-Alkoxy, ein- oder mehrfach mit Halogen substituiertes C₁₋₆-Alkyl oder C₁₋₆-Alkoxy substituiertes Phenyl, Pyridyl, Naphthyl, Chinolyl, Isochinolyl, Indenyl, Tetralinyl, Indolyl, Indazolyl, Benzothiazolyl oder Thienyl steht,
- R² und R³: für Wasserstoff, eine OH-Gruppe oder die Gruppe XR¹¹ stehen,
- X: für C₂₋₆-Alkyl, C₂₋₆-Alkenyl oder C₂₋₆-Alkinyl steht,
- R¹¹: unsubstituiertes oder gegebenenfalls ein- oder mehrfach mit Halogen, C₁₋₆-Alkoxy oder Hydroxy, substituiertes Phenyl, Pyrimidinyl oder Pyridyl bedeutet,
- R⁴, R⁵, R⁶ und R⁷: für Wasserstoff stehen,
- R⁸ und R¹⁰: für Wasserstoff oder C₁₋₆-Alkyl stehen, wobei R² und
R³ nicht gleichzeitig für Wasserstoff stehen und falls R² für eine OH - Gruppe steht, R³ nicht für Wasserstoff steht und falls R³ für eine OH-Gruppe steht, R² nicht für Wasserstoff steht, bedeuten, sowie deren Isomeren und Salze.

Die erfindungsgemäßen Verbindungen verhindern eine Phosphorylierung, d. h. bestimmte Tyrosinkinasen können selektiv inhibiert werden, wobei die persistente Angiogenese gestoppt werden kann. Somit wird beispielsweise das Wachstum und die Ausbreitung von Tumoren unterbunden.

Die erfindungsgemäßen Verbindungen der allgemeinen Formel I beinhalten auch die möglichen tautomeren Formen und umfassen die E- oder Z-Isomeren oder, falls ein chirales Zentrum vorhanden ist, auch die Racemate und Enantiomeren.

Die Verbindungen der Formel I sowie deren physiologisch verträglichen Salze sind auf Grund ihrer inhibitorischen Aktivität in Bezug auf Phosphorylierung des VEGF-Rezeptors als Arzneimittel verwendbar. Auf Grund ihres Wirkprofils eignen sich die erfindungsgemäßen Verbindungen zur Behandlung von Erkrankungen, die durch eine persistente Angiogenese hervorgerufen werden.

Da die Verbindungen der Formel I als Inhibitoren der Tyrosinkinase KDR und FLT identifiziert werden, eignen sie sich insbesondere zur Behandlung von solchen Krankheiten, die durch die über den VEGF-Rezeptor ausgelöste persistente Angiogenese oder eine Erhöhung der Gefäßpermeabilität hervorgerufen werden.

Gegenstand der vorliegenden Erfindung ist auch die Verwendung der erfindungsgemäßen Verbindungen als Inhibitoren der Tyrosinkinase KDR und FLT.

Gegenstand der vorliegenden Erfindung sind somit auch Arzneimittel zur Behandlung von Tumoren bzw. deren Verwendung.

Die erfindungsgemäßen Verbindungen können entweder alleine oder in Formulierung als Arzneimittel zur Behandlung von Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose und Verletzungen des Nervengewebes zum Einsatz kommen.

Bei der Behandlung von Verletzungen des Nervengewebes kann mit den erfindungsgemäßen Verbindungen eine schnelle Narbenbildung an den Verletzungsstellen verhindert werden, d. h. es wird verhindert, daß die Narbenbildung eintritt, bevor die Axone wieder Verbindung miteinander aufnehmen. Damit würde eine Rekonstruktion der Nervenverbindungen erleichtert.

Ferner kann mit den erfindungsgemäßen Verbindungen die Ascites-Bildung bei Patienten unterdrückt werden. Ebenso lassen sich VEGF bedingte Ödeme unterdrücken.
Derartige Arzneimittel, deren Formulierungen und Verwendungen sind ebenfalls Gegenstand der vorliegenden Erfindung.

Die Erfindung betrifft ferner die Verwendung der Verbindungen der allgemeinen Formel I, zur Herstellung eines Arzneimittels zur Behandlung von Tumoren, Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose und Verletzungen des Nervengewebes.

Zur Verwendung der Verbindungen der Formel I als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.
Die oben beschrieben Formulierungen und Darreichungsformen sind ebenfalls Gegenstand der vorliegenden Erfindung.
Die Herstellung der erfindungsgemäßen Verbindungen erfolgt nach an sich bekannten Methoden. Beispielsweise gelangt man zu Verbindungen der Formel I dadurch, daß man

### a) eine Verbindung der Formel II

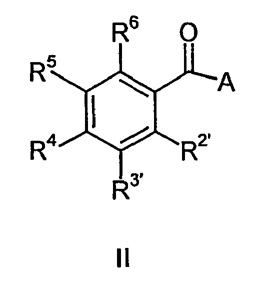

worin R⁴ bis R⁶ die obige Bedeutung haben und A Halogen oder OR¹¹ ist, wobei R¹¹ Wasserstoff, C₁₋₄-Alkyl oder C₁₋₄-Acyl und R^{2'} oder R^{3'} Wasserstoff, Aldehyd, Halogen oder OH, O-Triflat, O-Tosylat oder O-Mesylat bedeuten, zunächst R^{2'} oder R^{3'} in ein Alkenyl oder Alkinyl überführt, gegebenenfalls zum entsprechenden Alkan sättigt und dann COA in ein Amid überführt,
oder

### b) eine Verbindung der Formel III

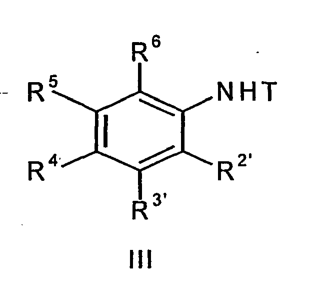

worin R⁴ bis R⁶ die obige Bedeutung haben und T eine Schutzgruppe bedeuten acyliert und dann gegebenenfalls die Ketogruppe zum Alkohol oder Alkan reduziert, die Schutzgruppe abspaltet, das Amin in ein Nitril überführt, das Nitril verseift und in ein Amid überführt.

Die Reihenfolge der Schritte kann in allen Fällen vertauscht werden.

Die Amidbildung erfolgt nach literaturbekannten Methoden.
Zur Amidbildung kann man von einem entsprechenden Ester ausgehen. Der Ester wird nach J. Org. Chem. 1995, 8414 mit Aluminiumtrimethyl und dem entsprechenden Amin in Lösungsmitteln wie Toluol bei Temperaturen von 0°C bis zum Siedepunkt des Lösungsmittels umgesetzt. Enthält das Molekül zwei Estergruppen, werden beide in das gleiche Amid überführt.

Beim Einsatz von Nitrilen statt des Esters erhält man unter analogen Bedingungen Amidine.

Zur Amidbildung stehen aber auch alle aus der Peptidchemie bekannten Verfahren zur Verfügung. Beispielsweise kann die entsprechende Säure in aprotischen polaren Lösungsmitteln wie zum Beispiel Dimethylformamid über eine aktiviertes Säurederivat, zum Beispiel erhältlich mit Hydroxybenzotriazol und einem Carbodiimid wie zum Beispiel Diisopropylcarbodiimid oder auch mit vorgebildeten Reagenzien wie zum Beispiel HATU (Chem. Comm. 1994, 201, oder BTU, bei Temperaturen zwischen 0°C und dem Siedepunkt des Lösungsmittels vorzugsweise bei 80°C mit dem Amin umgesetzt werden. Für die Amidbildung kann auch das Verfahren über das gemischte Säureanhydrid, Imidazolid oder Azid eingesetzt werden.

Zu den Salicylamiden gelangt man, wenn man das entsprechende Phenol in Gegenwart eines Friedel-Crafts-Katalysators wie Bortrichlorid mit Isocyanaten oder Isothiocyanaten in Lösungsmitteln wie beispielsweise Toluol bei Temperaturen von 0°C bis zum Siedepunkt des Lösungsmittels umsetzt.

Sollen verschiedene Amidgruppen in das Molekül eingeführt werden, muss beispielsweise die zweite Estergruppe nach der Erzeugung der ersten Amidgruppe in das Molekül eingeführt und dann amidiert werden oder man hat ein Molekül in dem eine Gruppe als Ester, die andere als Säure vorliegt und amidiert die beiden Gruppen nacheinander nach verschiedenen Methoden.

Thioamide sind aus den Anthranilamiden durch Umsetzung mit Diphosphadithianen nach Bull Soc.Chim.Belg. 87, 229,1978 oder durch Umsetzung mit Phosphorpentasulfid in Lösungsmitteln wie Pyridin oder auch ganz ohne Lösungsmittel bei Temperaturen von 0°C bis 200°C zu erhalten.

Die Produkte können auch einer elektrophilen aromatischen Substitutionen unterworfen werden. Die Substitution erfolgt dann an Verbindungen der Formel III in der ortho- oder para-Position zu der oder einer der Aminogruppe(n, an Verbindungen der Formel II in der meta Position) zur Carbonylgruppe. So kann durch Friedel-Crafts-Acylierung mit Säurechloriden in Gegenwart von Friedel-Crafts Katalysatoren wie zum Beispiel Aluminiumtrichlorid in Lösungsmitteln wie Nitromethan, Schwefelkohlenstoff, Methylenchlorid oder Nitrobenzol bei Temperaturen zwischen 0°C und dem Siedepunkt des Lösungsmittels vorzugsweise bei Raumtemperatur acyliert werden.
Es kann nach literaturbekannten Verfahren beispielsweise durch Nitriersäure, verschieden konzentrierte Salpetersäure ohne Lösungsmittel oder durch Metallnitrate wie beispielsweise Kupfer(II)nitrat oder Eisen(III)nitrat in polaren Lösungsmitteln wie Ethanol oder Eisessig oder auch in Acetanhydrid eine oder mehrere Nitrogruppen eingeführt werden.

Die Einführung von Halogenen erfolgt nach literaturbekannten Verfahren z.B. durch Umsetzung mit Brom, N-Brom- oder N-Jodsuccinimid oder Urotropinhydrotribromid in polaren Lösungsmitteln wie Tetrahydrofuran, Acetonitril, Methylenchlorid, Eisessig oder Dimethylformamid.

Die Reduktion der Nitrogruppe wird in polaren Lösungsmitteln bei Raumtemperatur oder erhöhter Temperatur durchgeführt. Als Katalysatoren für die Reduktion sind Metalle wie Raney-Nickel oder Edelmetallkatalysatoren wie Palladium oder Platin oder auch Palladiumhydroxid gegebenenfalls auf Trägern geeignet. Statt Wasserstoff können auch zum Beispiel Ammoniumformiat, Cyclohexen oder Hydrazin in bekannter Weise benutzt werden. Reduktionsmittel wie Zinn-II-chlorid oder Titan-(III)-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfalls unter Zusatz eines Lösungsmittels wie zum Beispiel Wasser, Methanol, Eisen/ Ammoniak etc. durchgeführt. Bei verlängerter Reaktionszeit kann bei dieser Variante eine Acylierung der Aminogruppe eintreten.

Wird eine Alkylierung einer Aminogruppe gewünscht, so kann nach üblichen Methoden - beispielsweise mit Alkylhalogeniden - oder nach der Mitsonubo Variante durch Umsetzung mit einem Alkohol in Gegenwart von beispielsweise Triphenylphosphin und Azodicarbonsäureester alkyliert werden. Man kann auch das Amin einer reduktiven Alkylierung mit Aldehyden oder Ketonen unterwerfen, wobei man in Gegenwart eines Reduktionsmittels wie beispielsweise Natriumcyanoborhydrid in einem geeigneten inerten Lösungsmittel wie zum Beispiel Ethanol bei Temperaturen von 0°C bis zum Siedepunkt des Lösungsmittels umsetzt. Wenn man von einer primären Aminogruppe ausgeht, so kann man gegebenenfalls nacheinander mit zwei verschiedenen Carbonylverbindungen umsetzen, wobei man gemischte Derivate erhält [Literatur z.B. Verardo et al. Synthesis (1993), 121; Synthesis (1991), 447; Kawaguchi, Synthesis (1985), 701; Micovic et al. Synthesis (1991), 1043]. Es kann vorteilhaft sein, zunächst die Schiffsche Base durch Umsetzung des Aldehyds mit dem Amin in Lösungsmitteln wie Ethanol oder Methanol, gegebenenfalls unter Zugabe von Hilfsstoffen wie Eisessig zu bilden und dann erst Reduktionsmittel wie z. B. Natriumcyanoborhydrid zuzusetzen.

Die Hydrierung von Alken-oder Alkingruppen im Molekül erfolgt in üblicher Weise beispielsweise durch katalytisch erregten Wasserstoff. Als Katalysatoren können Schwermetalle wie Palladium oder Platin, gegebenenfalls auf einem Träger oder Raney-Nickel benutzt werden. Als Lösungsmittel kommen Alkohole wie z.B. Ethanol in Frage. Es wird bei Temperaturen von 0° C bis zum Siedepunkt des Lösungsmittels und bei Drücken bis zu 20 Bar, vorzugsweise aber bei Raumtemperatur und Normaldruck gearbeitet. Durch die Verwendung von Katalysatoren, wie beispielsweise eines Lindlar-Katalysators lassen sich Dreifachbindungen zu Doppelbindungen partiell hydrieren, wobei vorzugsweise die Z-Form entsteht. Vorzugsweise wird diese Hydrierung in Pyridin als Lösungsmittel mit Palladium auf Calciumcarbonat als Katalysator vorgenommen. In gleicher Weise läßt sich die Z-Doppelbindung aus der Dreifachbindung durch Reduktion mit Diimin herstellen, beispielsweise nach R.M.Moriatry et al. Synth. Comm. 17, 703, 1987 herstellen.

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin, nach der Schotten-Baumann-Variante in wäßriger Lösung bei schwach alkalischem pH-Wert oder durch Umsetzung mit einem Anhydrid in Eisessig.

Eine Reduktion einer Ketogruppe erfolgt nach an sich bekannten Verfahren. So kann durch komplexe Metallhydride wie zum Beispiel Natriumborhydrid in Lösungsmitteln wie Methanol oder Isopropanol die Ketogruppe neben der Amidgruppe oder Estergruppe selektiv zum Alkohol reduziert werden. Eine Reduktion einer Ketogruppe zur Methylengruppe kann nach Clemmensen mit Zink in Salzsäure oder aber beispielsweise mit Silanen in Trifluoressigsäure erfolgen.

Die Einführung der Halogene Chlor, Brom, Jod oder der Azidogruppe über eine Aminogruppe kann beispielsweise auch nach Sandmeyer erfolgen, indem man die mit Nitriten intermediär gebildeten Diazoniumsalze mit Kupfer(I)chlorid oder Kupfer(I)bromid in Gegenwart der entsprechenden Säure wie Salzsäure oder Bromwasserstoffsäure oder mit Kaliumjodid umsetzt.

Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid. Die Entfernung der Aminogruppe kann entweder durch Umsetzung mit einem organischen Salpetrigsäureester in Tetrahydrofuran oder durch Diazotierung und reduktive Verkochung des Diazoniumsalzes beispielsweise mit phosphoriger Säure gegebenenfalls unter Zugabe von Kupfer (1) oxid bewerkstelligt werden.

Die Einführung von Fluor gelingt beispielsweise durch Balz-Schiemann-Reaktion des Diazoniumtetrafluorborates oder nach J. Fluor. Chem. 76,1996,59-62 durch Diazotierung i.G. von HFxPyridin und anschliessende Verkochung gegebenenfalls i.G. einer Fluoridionenquelle wie z.B. Tetrabutylammoniumfluorid.

Die Einführung der Azidogruppe gelingt nach Diazotierung durch Umsetzung mit Natriumazid bei Raumtemperatur.

Etherspaltungen werden nach literaturüblichen Verfahren durchgeführt. Dabei kann auch bei mehreren im Molekül vorhandenen Gruppen eine selektive Spaltung erreicht werden. Dabei wird der Ether beispielsweise mit Bortribromid in Lösungsmitteln wie Dichlormethan bei Temperaturen zwischen -100 °C bis zum Siedepunkt des Lösungsmittels vorzugsweise bei -78 °C behandelt. Es ist aber auch möglich, den Ether durch Natriumthiomethylat in Lösungsmitteln wie Dimethylformamid zu spalten. Die Temperatur kann zwischen Raumtemperatur und dem Siedepunkt des Lösungsmittels vorzugsweise bei 150°C liegen.

Die Einführung der Alkenylgruppe erfolgt mit den entsprechenden Vinylverbindungen unter den Bedingungen der Heck-Reaktion. Für die Einführung der Ethinylgruppen dient die Songashira-Reaktion.

Als Fluchtgruppe R^{2'} ist Halogene wie Fluor, Chlor, Brom, Jod oder O-Mesylat, O-Tosylat, O-Triflat oder O-Nonaflat geeignet. Die nucleophile Substitution zur Einführung von Ethinyl- bzw. Ethenylresten wird unter Katalyse von Übergangsmetallkomplexen wie Pd(0), z.B. Palladiumtetrakistriphenylphosphin oder Pd(2+), wie Palladium-bis-Tri-o-Tolylphosphin-dichlorid, Nickel(II) oder Nickel(0) nach literaturbekannten Methoden gegebenenfalls in Gegenwart einer Base und gegebenenfalls unter Cokatalyse eines Salzes wie zum Beispiel Kupfer (1) jodid oder Lithiumchlorid durchgeführt.

Als Nucleophile sind beispielsweise Vinyl- oder Ethinylverbindungen , zinnorganische Verbindungen oder zinkorganische Verbindungen geeignet. Die Umsetzung kann in polaren Lösungsmitteln wie Dimethylformamid, Dimethylacetamid, N-Methylpyrrolidon, Acetonitril, in Kohlenwasserstoffen wie Toluol oder in Ethern wie Tetrahydrofuran, Dimethoxyethan oder Diethylether vorgenommen werden. Als Basen sind anorganische Basen wie Alkali- oder Erdalkalihydroxide oder -hydrogencarbonate, -carbonate, -phosphate oder organische Basen wie cyclische, alicyclische und aromatische Amine, wie Pyridin, Triethylamin, DBU, Hünigbase geeignet, wobei in manchen Fällen Basen wie Diäthylamin oder Piperidin auch gleichzeitig Lösungsmittel sein können. Die Anwendung von Druck kann für die Reaktion förderlich sein.

Wird eine Trimethylsilylethinylgruppe eingeführt, so kann die Trimethylsilylgruppe durch Umsetzung mit Fluoriden wie beispielsweise Kaliumfluorid oder Tetrabutylammoniumfluorid in Lösungsmitteln wie Tetrahydrofuran, Methylenchlorid oder Acetonitril bei Temperaturen von 0°C bis zum Siedepunkt des Lösungsmittels,

Eine Alkenylgruppe kann aber auch durch Olefininierungsreaktionen wie z.B. die Petersonolefinierung, die Wittig- oder Wittig-Homer-Reaktion eingeführt werden. Dazu wird der Aldehyd mit dem vorher erzeugten Anion z.B. eines entsprechend substituierten Phosphoniumsalzes oder Phosphonsäureesters in Lösungsmitteln wie Toluol,Tetrahydrofuran, Diethylether oder Dimethoxyethan umgesetzt. Als Basen sind z.B. Alkalihydride, Alkaliamide, Alkalialkoholate wie beispielsweise Kaliumtertiärbutylat, Alkali- oder Erdalkalicarbonate oderhydroxide gegebenenfalls in Gegenwart von Phasentransferkatalysatoren wie z.B. Kronenäthern oder auch organische Basen wie Triethylamin, Diisopropylethylamin oder Diazabicycloundecan gegebenenfalls in Gegenwart von Salzen wie Lithiumbromid geeignet

Die Isomerengemische können nach üblichen Methoden wie beispielsweise Kristallisation, Chromatographie oder Salzbildung in die Enantiomeren bzw. EIZ-Isomeren aufgetrennt werden.

Die Herstellung der Salze erfolgt in üblicher Weise, indem man eine Lösung der Verbindung der Formel I mit der äquivalenten Menge oder einem Überschuß einer Base oder Säure, die gegebenenfalls in Lösung ist, versetzt und den Niederschlag abtrennt oder in üblicher Weise die Lösung aufarbeitet.

Soweit die Herstellung der Zwischenverbindungen nicht beschrieben wird, sind diese bekannt oder analog zu bekannten Verbindungen oder hier beschriebenen Verfahren herstellbar.

Zum Beispiel vom Stand der Technik können die folgenden Veröffentlichungen zitiert werden, in denen solche bekannten Verbindungen beschrieben werden :
Kang S.-K. et al., "Copper-Catalyzed Coupling of Polymer Bound Iodide with Organostannanes", Tetrahedron Letters, 1998, 39(19), pp. 3011-3012 ;
Srogl, Jiri et al., "Bio-organometallic Organosulfur Chemistry. Transition Metat-Catalysed Cross-Coupling Using Coenzyme M or Thioglycolic Acid as the Leaving Group", J. Amer. Chem. Soc., 1999, 121(40), pp. 9449-9450 ;
Shi, Chongsheng et al., "Biradicals from Thermolysis of N-[2-(1-Alkynyl)phenyl]-N-phenylcarbodiimides and their subsequent transformations to 6H-indolo [2,3-b] quinolines", J. Org. Chem., 1999, 64(3), pp. 925-932 ;
Ohno, Masatomi et al., "Phl(OAc)2-Promoted Rearrangement of the Hydroxyl Group : Ring Expansion of 4-Hydroxy-2-cyclobutenone to 2(5H)-furanone in Comparison with Ring Cleavage of the alpha-hydroxycycloalkanone to the omegaformyl ester", J. Org. Chem., 1999, 64(25), 8995-9000 ;
Justus Liebigs Ann. Chem., 463 ; 1928, Seite 267, (2-Phenylethyl-benzoic acid methyl ester) ;
Can. J. Chem., Bd. 73, Nr, 10, 1995, Seiten 1660-1665, (2-Styryl-benzoic acid methyl ester) ;
und
Magn. Reson. Chem., Bd. 27, Nr. 6, 1989, Seiten 585-591 (3-benzyl-benzoic acid methyl ester).

Die beschriebenen Zwischenverbindungen sind besonders zur Herstellung der erfindungsgemäßen Benzoesäureamide geeignet.

Besonders geeignet sind solche Zwischenverbindungen der allgemeinen Formel II 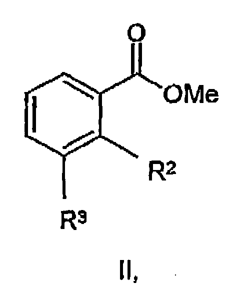 in der
- R² und R³: Wasserstoff oder die Gruppe XR¹¹,
- X: C₁₋₆-Alkyl, C₂₋₆-Alkenyl oder C₂₋₆-Alkinyl,
- R¹¹: gegebenenfalls dmit C₁₋₆-Alkoxy substituiertes Phenyl oder Pyridyl bedeuten, wobei R² und R³ nicht gleichzeitig für Wasserstoff stehen, sowie deren Isomeren und Salze.

Die Zwischenprodukte sind teilweise selbst aktiv und können somit ebenfalls zur Herstellung eines Arzneimittels zur Behandlung von Tumoren, Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose und Verletzungen des Nervengewebes zum Einsatz kommen.

Die nachfolgenden Beispiele erläutern die Herstellung der erfindungsgemäßen Verbindungen, ohne den Umfang der beanspruchten Verbindungen auf diese Beispiele zu beschränken.

### Beispiel 1.0

Herstellung von (N-4-Chlorphenyl)-2-(4-pyridylethyl)benzoesäureamid 105mg 2-(4-Pyridylethyl)benzoesäuremethylester werden in 7,5ml Toluol mit 56mg 4-Chloranilin versetzt, auf 4°C abgekühlt und unter Argon und Feuchtigkeitsausschluß mit 0,22ml Trimethylaluminium (2m Lösung in Hexan) versetzt. Anschließend wurde das Gemisch 2h auf 120°C Badtemperatur erwärmt. Nach Abkühlen wird mit 30ml einer verdünnten NatriumbicarbonatLösung versetzt und zweimal mit je 25ml Essigester extrahiert. Die organische Phase wird mit Wasser gewaschen, getrocknet filtriert und eingeengt. Der Rückstand wird über Kieselgel mit Essigester:Cyclohexan=1:1 als Elutionsmittel chromatographiert. Man erhält 133mg (89% derTheorie) (N-4-Chlorphenyl)-2-(4-pyridylethyl)-benzoesäureamid als Öl.

In analoger Verfahrensweise zu Beispiel 1.0 werden hergestellt:

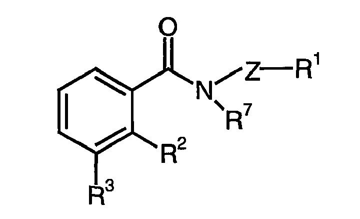

| **Beispiel** | **-Z-R¹** | **R⁷** | **R²** | **R³** | **Schmelzpunkt °C** |
|---|---|---|---|---|---|
| 1.1 | 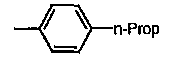 | H | 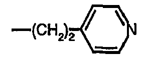 | H | |
| 1.2 | 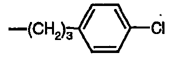 | H | 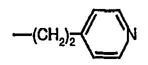 | H | |
| 1.3 | 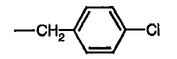 | H | 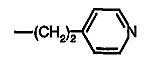 | H | 98-99 |
| 1.4 | 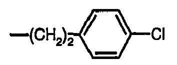 | H | 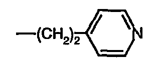 | H | Öl |
| 1.5 | 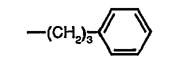 | H | 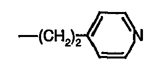 | H | Öl |
| 1.6 | 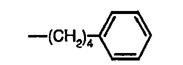 | H | 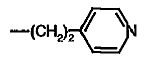 | H | Öl |
| 1.7 | 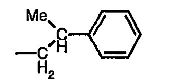 | H | 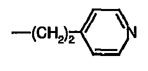 | H | |
| 1.8 | 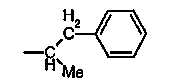 | H | 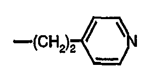 | H | Öl |
| 1.9 | 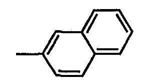 | H | 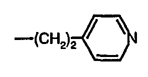 | H | |
| 1.10 | 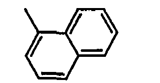 | H | 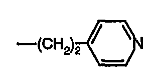 | H | |
| 1.11 | 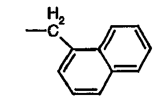 | H | 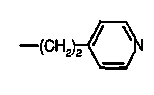 | H | Öl |
| 1.12 | 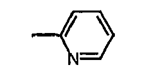 | H | 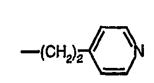 | H | |
| 1.13 | 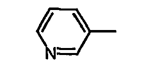 | H | 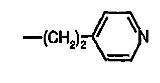 | H | |
| 1.14 | 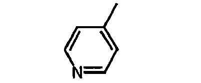 | H | 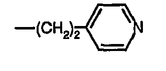 | H | |
| 1.15 | 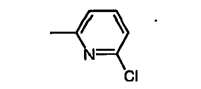 | H | 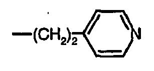 | H | |
| 1.16 | 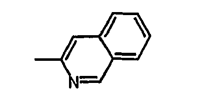 | H | 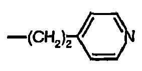 | H | |
| 1.17 | 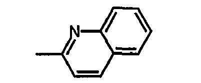 | H | 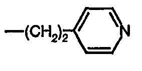 | H | Öl |
| 1.18 | 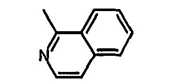 | H | 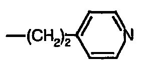 | H | |
| 1.19 | 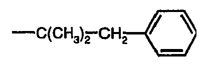 | H | 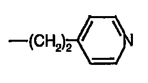 | H | |
| 1.20 | 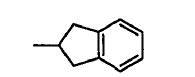 | H | 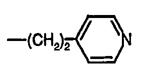 | H | 121-122 |
| 1.21 | 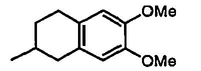 | H | 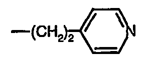 | H | |
| 1.22 | 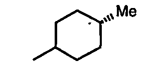 | H | 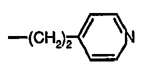 | H | |
| 1.23 | 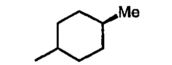 | H | 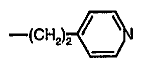 | H | |
| 1.24 | 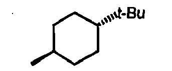 | H | 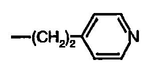 | H | 130-131 |
| 1.25 | 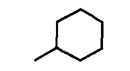 | H | 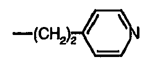 | H | |
| 1.26 | 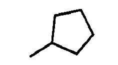 | H | 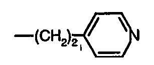 | H | |
| 1.27 | 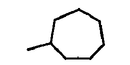 | H | 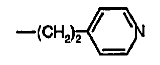 | H | |
| 1.28 | 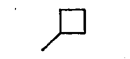 | H | 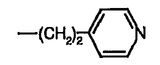 | H | |
| 1.29 | 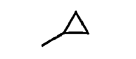 | H | 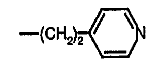 | H | |
| 1.30 | 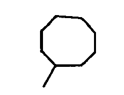 | H | 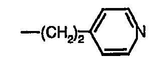 | H | |
| 1.31 | 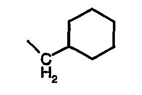 | H | 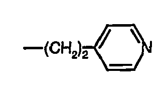 | H | 105-107 |
| 1.32 | -(CH₂)₁₁-Me | H | 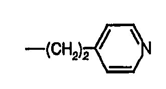 | H | |
| 1.33 | -(CH₂)₆-Me | H | 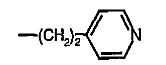 | H | |
| 1.34 | 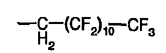 | H | 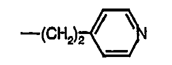 | H | |
| 1.35 | -(CH₂)₃-C₃ | H | 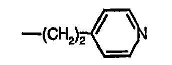 | H | |
| 1.36 | -(CH₂)₂-t-Bu | H | 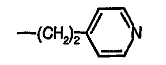 | H | |
| 1.37 | -(CH₂)₂-i-Prop | H | 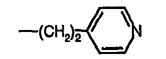 | H | |
| 1.38 | 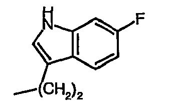 | H | 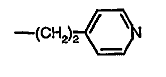 | H | |
| 1.39 | 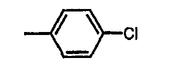 | H | 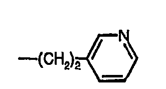 | H | 105,5 |
| 1.40 | 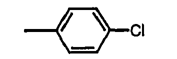 | H | 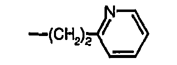 | H | 136,2 |
| 1.41 | 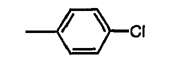 | H | 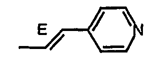 | H | Öl |
| 1.42 | 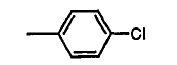 | H | 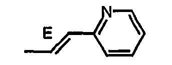 | H | 181,2 |
| 1.43 | 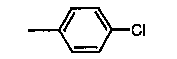 | H | 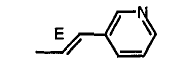 | H | Öl |
| 1.44 | 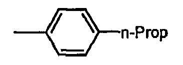 | H | 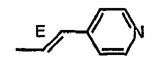 | H | |
| 1.45 | 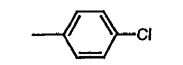 | H | 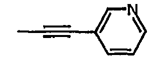 | H | 156,4 |
| 1.46 | 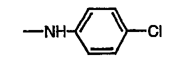 | H | 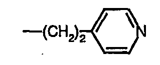 | H | |
| 1.47 | 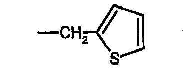 | H | 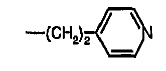 | H | |
| 1.48 | 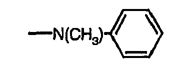 | H | 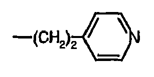 | H | |
| 1.49 | 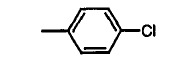 | H | H | 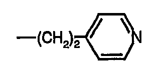 | 191,6 |
| 1.50 | 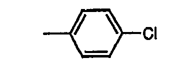 | H | H | 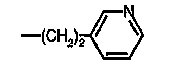 | 106,5 |
| 1.51 | 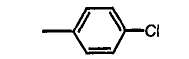 | H | H | 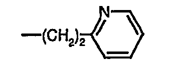 | 128,5 |
| 1.52 | 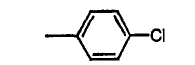 | H | H | 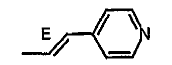 | 191,5 |
| 1.53 | 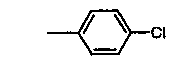 | H | H | 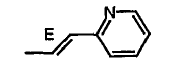 | 212,7 |
| 1.54 | 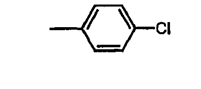 | H | H | 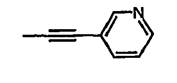 | 179 |
| 1.55 | 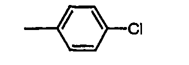 | H | H | 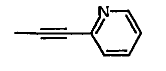 | >300 |
| 1.56 | 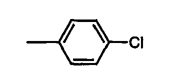 | H | H | 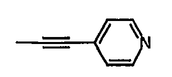 | 163,5 |
| 1.57 | 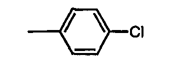 | H | 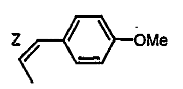 | H | 137,9 |
| 1.58 | 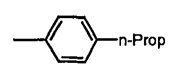 | H | 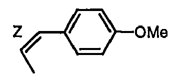 | H | 115-118 |
| 1.59 | 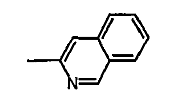 | H | 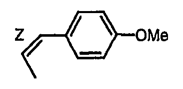 | H | 151-153 |
| 1.60 | 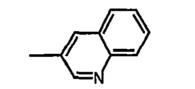 | H | 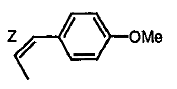 | H | |
| 1.61 | 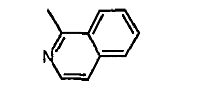 | H | 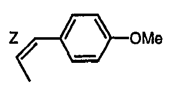 | H | |
| 1.62 | 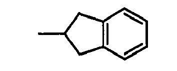 | H | 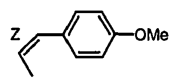 | H | |
| 1.63 | 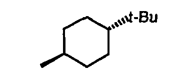 | H | 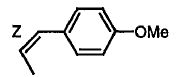 | H | |
| 1.64 | n-Heptyl | H | 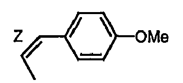 | H | Öl |
| 1.65 | 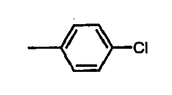 | H | 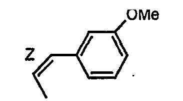 | H | 120,2 |
| 1.66 | 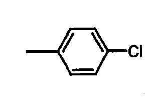 | H | 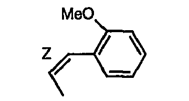 | H | 108,6 |
| 1.67 | 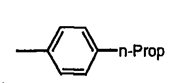 | H | 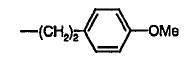 | H | 113,7 |
| 1.68 | 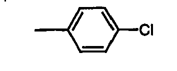 | H | 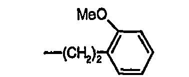 | H | |
| 1.69 | 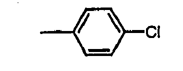 | H | 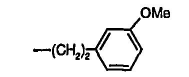 | H | |
| 1.70 | 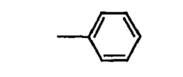 | H | 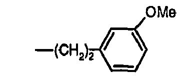 | H | |
| 1.71 | 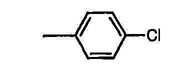 | H | 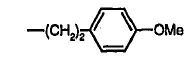 | H | |
| 1.72 | 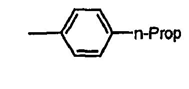 | H | H | 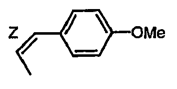 | |
| 1.73 | 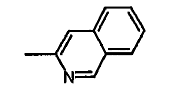 | H | H | 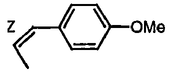 | |
| 1.74 | 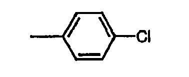 | H | H | 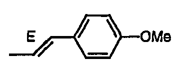 | 204,7 |
| 1.75 | 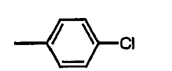 | H | H | 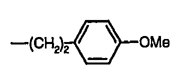 | >300 |
| 1.76 | 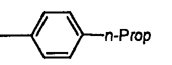 | H | H | 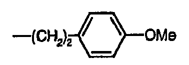 | |
| 1.77 | 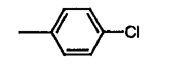 | H | H | 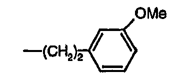 | 126,6 |
| 1.78 | 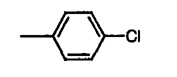 | H | H | 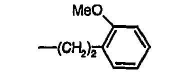 | 133,2 |
| 1.79 | 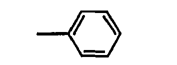 | H | H | 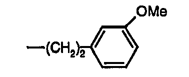 | 139,5 |
| 1.80 | 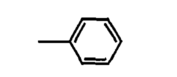 | H | H | 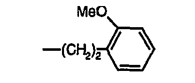 | 126,3 |
| 1.81 | 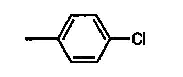 | H | 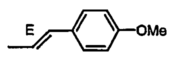 | H | |
| 1.82 | 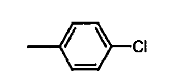 | H | 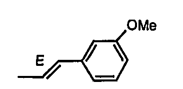 | H | 163,4 |
| 1.83 | 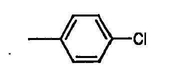 | H | 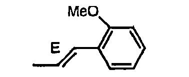 | H | 185,4 |
| 1.84 | 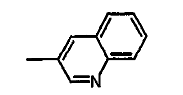 | H | 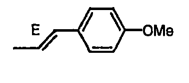 | H | Öl |
| 1.85 | 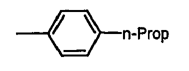 | H | 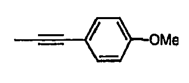 | H | |
| 1.86 | 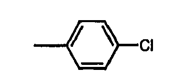 | H | 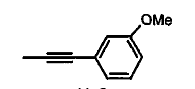 | H | 136,8 |
| 1.87 | 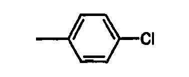 | H | 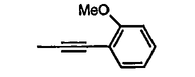 | H | 131,1 |
| 1.88 | 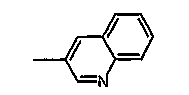 | H | 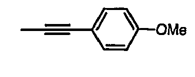 | H | 140,6 |
| 1.89 | 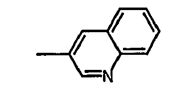 | H | 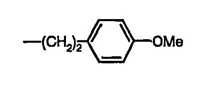 | H | Öl |
| 1.90 | 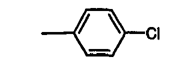 | H | H | 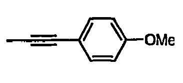 | 194,6 |
| 1.91 | 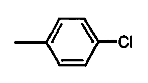 | H | H | 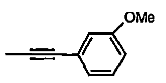 | 188,9 |
| 1.92 | 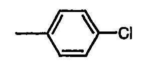 | H | H | 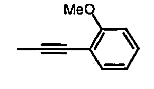 | |
| 1.93 | 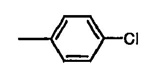 | H | 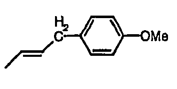 | H | 146,4 |
| 1.94 | 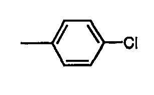 | H | 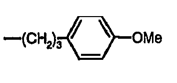 | H | |

### Beispiel 2.0

### Herstellung von an E- N-4-Chlorphenyl- 3-(2-pyridylethenyl)benzoesäureamid

179mg N-4-Chlorphenyl- 3-(2-pyridylethinyl)benzoesäureamid werden in 7ml Pyridin mit 20mg Palladium auf Calciumcarbonat (5%ig) versetzt und 1,5h unter Normalwasserstoffdruck bei Raumtemperatur hydriert. Nach Absaugen vom Katalysator über Kieselgur wird das Filtrat eingeengt. Der Rückstand wird über Kieselgel mit Essigester:Hexan=1:1 als Elutionsmittel chromatographiert. Man erhält 123mg (68% der Theorie) an (Z)- N-4-Chlorphenyl- 3-(2-pyridylethenyl)-benzoesäureamid als Öl.

In analoger Verfahrensweise zu Beispiel 2.0 werden auch folgende Verbindungen hergestellt:

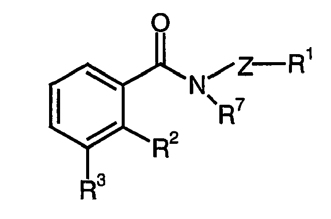

| **Beispiel** | **-Z-R¹** | **R⁷** | **R²** | **R³** | **Schmelzpkt. °C** |
|---|---|---|---|---|---|
| 2.1 | 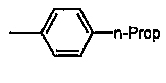 | H | H | 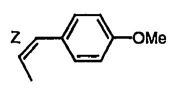 | |
| 2.2 | 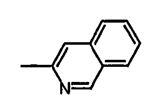 | H | H | 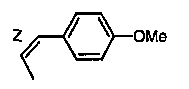 | |
| 2.3 | 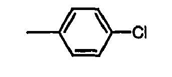 | H | H | 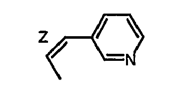 | 120,1 |
| 2.4 | 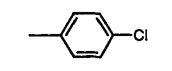 | H | H | 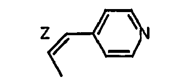 | 157,9 |
| 2.5 | 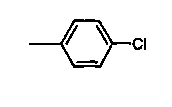 | H | H | 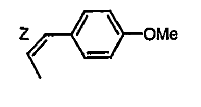 | 95,2 |
| 2.6 | 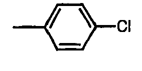 | H | H | 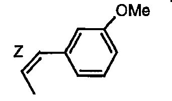 | 116,2 |
| 2.7 | 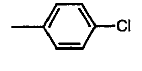 | H | H | 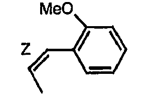 | 123 |

### Beispiel 3.0

### Herstellung von (E)- N-4-Chlorphenyl- 3-(2-pyridylethenyl)benzoesäureamid

120mg (Z)- N-4-Chlorphenyl- 3-(2-pyridylethenyl)benzoesäureamid werden in Toluol mit Jod versetzt und 7h am Rückfluß gekocht. Nach Einengen wird der Rückstand über Kieselgel mit Essigester:Hexan=1:1 als Elutionsmittel chromatographiert. Man erhält 60mg (50% d.Th.) an (E)- N-4-Chlorphenyl- 3-(2-pyridylethenyl)benzoesäureamid vom Schmelzpunkt 212,7°C.

In analoger Weise werden hergestellt:

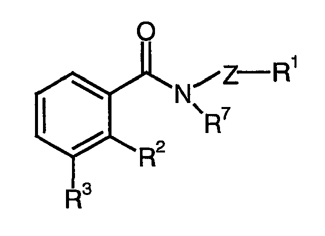

| **Beispiel** | **-Z-R¹** | **R⁷** | **R²** | **R³** | **Schmelzpkt. °C** |
|---|---|---|---|---|---|
| 3.1 | 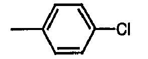 | H | H | 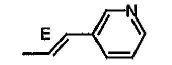 | 173,2 |
| 3.2 | 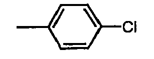 | H | H | 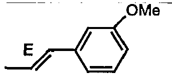 | 146,9 |
| 3.3 | 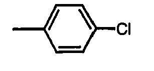 | H | H | 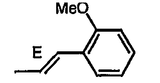 | 178 |

### Beispiel 4.0

### Herstellung von N,-(4-Chlorphenyl)-2-(3-[4-hydroxyphenyl)propyl)]benzoesäure-amid

90mg N-(4-Chlorphenyl)-2-(3-[4-methoxyphenyl)propyl)]benzoesäureamid werden in 8ml Methylenchlorid bei -78°C tropfenweise mit 1,2ml Bortribromid versetzt und nach beendeter Zugabe über Nacht bei Raumtemperatur gerührt. Anschließend wird mit Wasser versetzt, das Methylenchlorid am Vakuum abdestilliert und das Wasser mit Essigester ausgeschüttelt. Die Essigesterphase wird eingeengt und der Rückstand über Kieselgel mit Hexan:Essigester=8:2 als Elutionsmittel chromatographiert. Man erhält 24mg (28% der Theorie) an N-(4-Chlorphenyl)-2-(3-[4-hydroxyphenyl)propyl)]-benzoesäureamid

In analoger Verfahrensweise werden hergestellt:

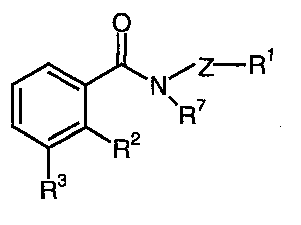

| **Beispiel** | **-Z-R¹** | **R⁷** | **R²** | **R³** | **Schmelzpkt. °C** |
|---|---|---|---|---|---|
| 4.1 | 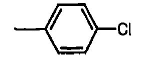 | H | 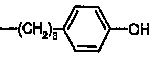 | H | |
| 4.2 | 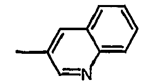 | H | 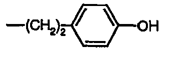 | H | |
| 4.3 | 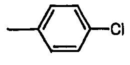 | H | H | 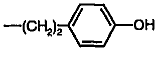 | |
| 4.4 | 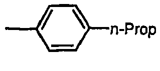 | H | 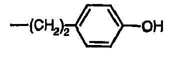 | H | |
| 4.5 | 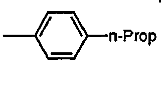 | H | H | 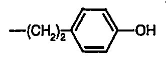 | |
| 4.6 | 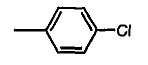 | H | 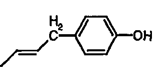 | H | |
| 4.7 | 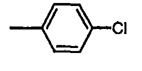 | H | OH | 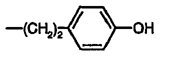 | 164,1 |
| 4.8 | 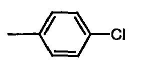 | H | OH | 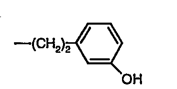 | 115,3 |
| 4.9 | 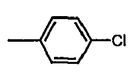 | H | OH | 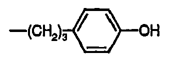 | 137,4 |
| 4.10 | 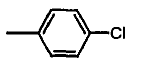 | H | OH | 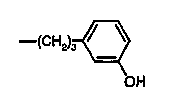 | Öl |
| 4.11 | 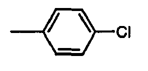 | H | OH | 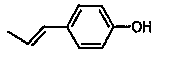 | 203,7 |
| 4.12 | 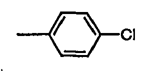 | H | OH | 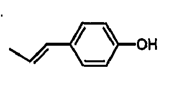 | |

### Beispiel 5.0

### Herstellung von N-(4-Chlorphenyl)-3-(4-methoxystyryl)salicylsäureamid

904mg 4-Methoxy-2'-hydroxystyrol werden in 40ml Toluol vorgelegt und bei 4°C mit 4ml einer Lösung von Bortrichlorid (1 molar in Hexan) versetzt. Es wird dann bei Raumtemperatur für 1h gerührt, mit 614mg 4-Chlorphenylisocyanat versetzt und 1,5h auf 120°C erwärmt. Anschließend wird mit 5ml Methanol versetzt und eingeengt. Der Rückstand wird zweimal über Kieselgel zunächst mit Essigester:Hexan=1:1 und ein zweites Mal mit Toluol:Essigester=100:3,5 als Elutionsmittel chromatographiert. Man erhält 150mg (10% d.Th.) an N-(4-Chlorphenyl)-3-(4-methoxystyryl)salicylsäureamid als Öl. In analoger Verfahrensweise werden aus den entsprechenden Ausgangsmaterialien hergestellt:

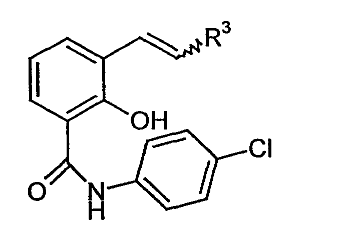

| **Beispiel** | **R³** | **Schmelzpkt. °C** |
|---|---|---|
| 5.1 | 3-MeO-Ph | 116,3 |
| 5.2 | 3-MeO-Ph-CH₂ | |
| 5.3 | 4-MeO-Ph-CH₂ | 163,6 |

### Herstellung der Zwischenverbindungen

### Beispiel Z - 5.0

### Herstellung von 4-Methoxy-2'-hydroxystyrol

2,44g Salicylaldehyd werden in 200ml Toluol zunächst mit 12,5g 4-Methoxybenzyltriphenylphosphoniumchlorid versetzt. Unter Eiskühlung werden dann 2,24g Kalium-tert.-butylat zugefügt. Danach wird zunächst 1 h bei dieser Temperatur und anschliessend 3,5h bei Raumtemperatur gerührt. Nach Versetzen mit 100ml Wasser und Ansäuern mit 1 N-Salzsäure wird dreimal mit 50ml Essigester extrahiert. Die gesammelte organische Phase wird mit gesättigter Natriumchloridlösung gewaschen, getrocknet, filtriert und eingeengt. Der Rückstand wird über Kieselgel mit Essigester:Hexan=2:8 als Elutionsmittel chromatographiert. Man erhält 3,1g (68% der Theorie) an 4-Methoxy-2'-hydroxystyrol

In analoger Verfahrensweise werden auch folgende Verbindungen hergestellt

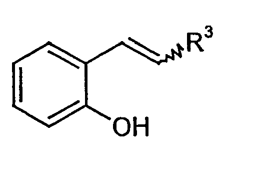

| **Beispiel** | **R³** | **Schmelzpunkt °C** |
|---|---|---|
| Z-5.1 | 3-MeO-Ph | Öl |
| Z-5.2 | 3-MeO-Ph-CH₂ | Öl |
| Z-5.3 | 4-MeO-Ph-CH₂ | Öl |

### Beispiel 6.0

### Herstellung von N-(4-Chlorphenyl)-3-(4-methoxyphenethyl) salicylsäureamid

813mg 4-Methoxy-2'-hydroxy 1,2-diphenylethan werden analog Beispiel Z-5.0 umgesetzt. Der nach der dort beschriebenen Aufarbeitung erhaltene Rückstand wird über Kieselgel mit Essigester:Hexan=1:1 als Elutionsmittel chromatographiert und die entsprechenden Fraktionen eingeengt und mit Essigester/Hexan kristallin gerührt. Man erhält 375mg (27,6% der Theorie) an N-(4-Chlorphenyl)-3-(4-methoxyphenylethyl)salicylsäureamid vom Schmelzpunkt 141°C.

In analoger Verfahrensweise werden auch folgende Verbindungen hergestellt:

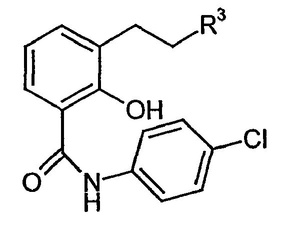

| **Beispiel** | **R³** | **Schmelzpunkt °C** |
|---|---|---|
| 6.1 | 3-MeO-Ph | 130,2 |
| 6.2 | 3-MeO-Ph-CH₂ | Öl |
| 6.3 | 4-MeO-Ph-CH₂ | 158 |

### Herstellung der Zwischenverbindungen

### Beispiel Z - 6.0

### Herstellung von 4-Methoxy-2'-hydroxy -1,2-diphenylethan

905mg 4-Methoxy-2'-hydroxystyrol werden in 50ml Ethanol mit 1,3g Palladium auf Kohlenstoff (10) versetzt und 70 min bei Raumtemperatur unter Wasserstoffnormaldruck hydriert Nach Absaugen vom Katalysator und Einengen erhält man 880mg 4-Methoxy-2'-hydroxy 1,2-diphenylethan.

In analoger Verfahrensweise werden auch folgende Verbindungen hergestellt:

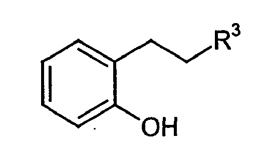

| | | |
|---|---|---|
| **Beispiel** | **R³** | **Schmelzpunkt °C** |
| Z-6.1 | 3-MeO-Ph | Öl |
| Z-6.2 | 3-MeO-Ph-CH₂ | Öl |
| Z-6.3 | 4-MeO-Ph-CH₂ | |

### Beispiel 7.0

### Herstellung der Zwischenprodukte

Die nachfolgenden Beispiele erläutert die Herstellung der erfindungsgemäßen Zwischenprodukte, die zur Herstellung der erfindungsgemäßen Verbindungen der allgemeinen Formel I besonders geignet sind, ohne die Erfindung auf diese Beispiele zu beschränken.

### Methode A

### Herstellung von 2-(4-Pyridylethenyl)benzoesäuremethylester

Unter Argon wird ein Gemisch von 2,10 g 2-Jodbenzoesäuremethylester und 0,97g 4-Vinylpyridin in 24 ml Dimethylformamid mit 1,04g Triethylamin und 40mg Palladium(II)acetat sowie 24mg Tri-o-tolylphosphin versetzt und 5h in einem Glasdruckbehälter auf 100°C erwärmt. Nach Einengen am Vakuum wird der Rückstand über Kieselgel mit Hexan:Essigester=1:1 als Elutionsmittel chromatographiert.
Man erhält 1,8g (94% der Theorie) an 2-(4-Pyridylethenyl)-benzoesäure-methylester.

### Methode B

### Herstellung von 2-(4-Pyridylethinyl)benzoesäuremethylester

2,10 g 2-Jodbenzoesäuremethylester werden in 25ml Dimethylformamid unter Argon mit 2,94g Triethylamin, 179mg Bis(triphenylphosphin)palladium(II)chlorid, 111mg Kupfer(l)jodid und 900mg 4-Ethinylpyridin versetzt und in einem Glasdruckgefäß für 3,5h auf 80°C Badtemperatur erwärmt. Nach Einengen am Vakuum wird der Rückstand über Kieselgel mit Hexan:Aceton=1:1 als Elutionsmittel chromatographiert.
Man erhält 1,08g (45% der Theorie) an 2-(4-Pyridylethinyl)-benzoesäuremethylester

### Methode C

### Herstellung von 2-(4-Pyridylethyl)benzoesäuremethylester

237mg 2-(4-Pyridylethinyl)benzoesäuremethylester werden in 30ml Ethanol mit 200mg Palladium auf Kohlenstoff (10%ig) versetzt und bei Normaldruck und Raumtemperatur 20 Minuten hydriert. Anschliessend wird vom Katalysator über Kieselgur abgesaugt und das Filtrat eingeengt. Man erhält 220mg 2-(4-Pyridylethyl)benzoesäuremethylester.
Statt der Ethinylverbindung kann auch die entsprechende Rthenylverbindung eingesetzt werden.

### Methode D

Nach der in Beispiel 2.0 beschriebenen Methode lassen sich auch die entsprechenden Ester in die Z-Verbindungen umwandeln.

### Methode E

Nach der in Beispiel 3.0 beschriebenen Methode lassen sich auch bei den Estern aus der entsprechenden Z-Verbindung die E-Verbindungen herstellen

### Methode F

Nach der in Methode B beschriebenen Methode lässt sich auch aus 2-Jodbenzoesäureethylester mit Ethinyltrimethylsilan in 83% Ausbeute 2-Trimethylsilylethinylbenzoesäuremethylester herstellen.

### Methode G

464mg 2- Trimethylsilylethinylbenzoesäuremethylester werden in 15ml absolutem Methylenchlorid mit 2,75ml Tetrabutylammoniumfluorid (1 M in Tetrahydrofuran) versetzt und 2,5 h bei Raumtemperatur gerührt. Nach Waschen mit verdünntem Ammoniak wird die organische Phase getrocknet, filtriert und eingeengt und ohne weitere Reinigung in die nächste Stufe eingesetzt.

### Methode H

440mg 2- Ethinylbenzoesäuremethylester werden mit 1,94g 3-Jodanisol nach Methode B umgesetzt und ergeben nach Säulenchromatographie über Kieselgel mit Essigester:Hexan=2:8 als Elutionsmittel 680mg (55,3% d.Th.) an 2-Carbetoxymethyl-3'-methoxydiphenylacetylen.

In analoger Verfahrensweise werden auch folgende Verbindungen hergestellt:

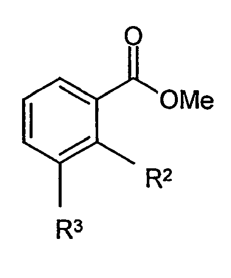

| **Beispiel** | **R²** | **R³** | **Methode** |
|---|---|---|---|
| 7.0 | 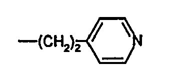 | H | C |
| 7.1 | 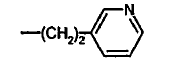 | H | C |
| 7.2 | 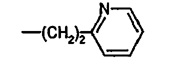 | H | C |
| 7.3 | 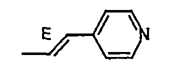 | H | A |
| 7.4 | 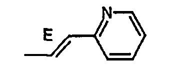 | H | A |
| 7.5 | 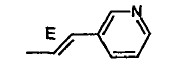 | H | E |
| 7.6 | 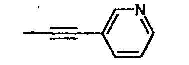 | H | B |
| 7.7 | 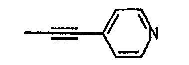 | H | B |
| 7.8 | 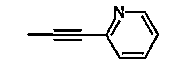 | H | B |
| 7.9 | H | 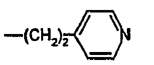 | C |
| 7.10 | H | 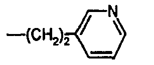 | C |
| 7.11 | H | 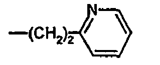 | C |
| 7.12 | H | 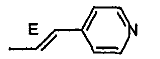 | A |
| 7.13 | H | 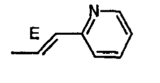 | A |
| 7.14 | H | 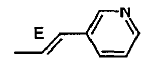 | E |
| 7.15 | H | 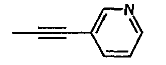 | B |
| 7.16 | H | 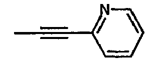 | B |
| 7.17 | H | 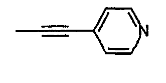 | B |
| 7.18 | H | 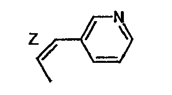 | D |
| 7.19 | 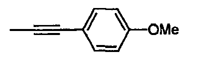 | H | B |
| 7.20 | 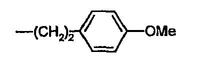 | H | C |
| 7.21 | 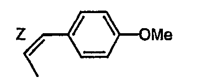 | H | D |
| 7.22 | 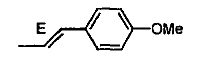 | H | A |
| 7.23 | 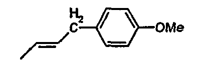 | H | A |
| 7.24 | 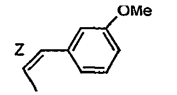 | H | D |
| 7.25 | 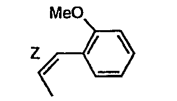 | H | D |
| 7.26 | 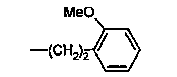 | H | C |
| 7.27 | 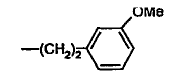 | H | C |
| 7.28 | 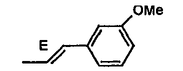 | H | E |
| 7.29 | 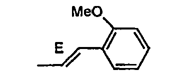 | H | A |
| 7.30 | 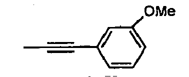 | H | F-H |
| 7.31 | 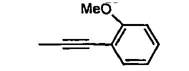 | H | B |
| 7.32 | 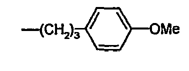 | H | C |
| 7.33 | H | 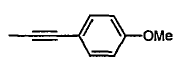 | B |
| 7.34 | H | 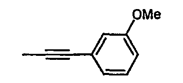 | F-H |
| 7.35 | H | 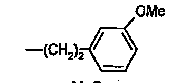 | C |
| 7.36 | H | 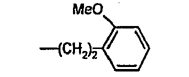 | C |
| 7.37 | H | 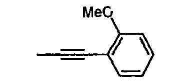 | F-H |
| 7.38 | H | 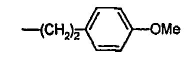 | C |

Die nachfolgenden Anwendungsbeispiele erläutern die biologische Wirkung und Verwendung der erfindungsgemäßen Verbindungen ohne diese auf die Beispiele zu beschränken.

**Für die Versuche benötigte Lösungen**
Stammlösungen
Stammlösung A: 3mM ATP in Wasser pH 7,0 (-70°C)
Stammlösung B: g-33P-ATP 1mCi/ 100µl
Stammlösung C: poly-(Glu4Tyr) 10mg/ ml in Wasser

Lösung für Verdünnungen
- Substratlösemittel:: 10mM DTT, 10 mM Manganchlorid, 100 mM Magnesiumchlorid
- Enzymlösung:: 120 mM Tris/ HCI, pH 7,5, 10 µM Natriumvanadiumoxid

### Anwendungsbeispiel 1

### Hemmung der KDR- und FLT-1 Kinaseaktivität in Gegenwart der erfindungsgemäßen Verbindungen

In einer spitz zulaufenden Mikrotiterplatte (ohne Proteinbindung) werden 10 µl Substratmix (10µl Vol ATP Stammlösung A + 25µCi g-33P-ATP (ca. 2,5µl der Stammlösung B) + 30µl poly-(Glu4Tyr) Stammlösung C + 1,21ml Substratlösemittel), 10 µl Hemmstofflösung (Substanzen entsprechend den Verdünnungen, als Kontrolle 3% DMSO in Substratlösemittel) und 10 µl Enzymlösung (11,25µg Enzymstammlösung (KDR oder FLT-1 Kinase) werden bei 4°C in 1,25ml Enzymlösung verdünnt) gegeben. Es wird gründlich durchgemischt und bei 10 Minuten Raumtemperatur inkubiert. Anschließend gibt man 10µl Stop-Lösung (250mM EDTA, pH 7,0) zu, mischt und überträgt 10 µl der Lösung auf einen P 81 Phosphozellulosefilter. Anschließend wird mehrfach in 0,1 M Phosphorsäure gewaschen. Das Filterpapier wird getrocknet, mit Meltilex beschichtet und im Microbetazähler gemessen.

Die IC50-Werte bestimmen sich aus der Inhibitorkonzentration, die notwendig ist, um den Phosphateinbau auf 50% des ungehemmten Einbaus nach Abzug des Leerwertes (EDTA gestoppte Reaktion) zu hemmen.

Die Ergebnisse der Kinase-Inhibition IC50 in µM sind in der nachfolgenden Tabelle dargestellt:

| **Beispiel-Nr.** | **VEGFR I (FLT)** | **VEGFR II (KDR)** |
|---|---|---|
| 1.60 | 2 | 0,5 |
| 1.31 | 0,2 | 0,4 |
| 1.89 | 2 | 0,3 |
| 1,54 | 0,05 | 0,5 |
| 1,57 | 0,2 | 0,2 |
| 1.64 | 0,2 | 0,3 |
| 1.67 | KH | 5 |
| 1.1 | 0,2 | 0,2 |

| | | |
|---|---|---|
| KH= keine Hemmung | | |

## Patentansprüche

1. Verbindungen der allgemeinen Formel I 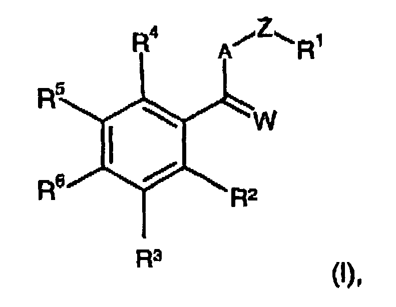 in der
A für die Gruppe =NR⁷ steht,
W für Sauerstoff oder zwei Wasserstoffatome steht,
Z für eine Bindung, die Gruppe =NR¹⁰ oder verzweigtes oder unverzweigtes C₁₋₁₂-Alkyl steht,
R¹ für verzweigtes oder unverzweigtes C₁₋₅-Alkyl, oder gegebenenfalls ein- oder mehrfach mit Halogen oder C₁₋₆-Alkyl substituiertes C₃₋₁₀-Cycloalkyl oder unsubstituiertes oder gegebenenfalls ein- oder mehrfach mit Halogen, C₁₋₆-Alkyl, C₁₋₆-Alkoxy, ein- oder mehrfach mit Halogen substituiertes C₁₋₆-Alkyl oder C₁₋₆-Alkoxy substituiertes Phenyl, Pyridyl, Naphthyl, Chinolyl, Isochinolyl, Indenyl, Tetralinyl, Indolyl, Thienyl, Indazolyl oder Benzothiazolyl steht,
R² und R³ für Wasserstoff, eine OH-Gruppe oder die Gruppe XR¹¹ stehen,
X für C₂₋₆-Alkyl, C₂₋₆-Alkenyl oder C₂₋₆-Alkinyl steht,
R¹¹ unsubstituiertes oder gegebenenfalls ein- oder mehrfach mit Halogen, C₁₋₆-Alkoxy oder Hydroxy, substituiertes Phenyl, Pyrimidinyl oder Pyridyl bedeutet,
R⁴, R⁵, R⁶und R⁷ für Wasserstoff stehen,
R⁸ und R¹⁰ für Wasserstoff oder C₁₋₈-Alkyl stehen, wobei R² und
R³ nicht gleichzeitig für Wasserstoff stehen und falls R² für eine OH - Gruppe steht, R³ nicht für Wasserstoff steht und falls R³ für eine OH-Gruppe steht, R² nicht für Wasserstoff steht, bedeuten, sowie deren Isomeren und Salze.

2. Verwendung der Verbindungen der allgemeinen Formel I, gemäß Anspruch 1, zur Herstellung eines Arzneimittels zur Behandlung von Tumoren, Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose und Verletzungen des Nervengewebes.

3. Arzneimittel, enthaltend mindestens eine Verbindung gemäß Anspruch 1.

4. Arzneimittel gemäß Anspruch 3, zur Behandlung von Tumoren, Psoriasis, Arthritis, wie rheumatoide Arthritis, Hämangioma, Angiofribroma, Augenerkrsnkungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose und Verletzungen des Nervengewebes,

5. Verbindungen gemäß Anspruch 1 mit geeigneten Formulierungs und Trägerstoffen.

6. Verwendung der Verbindungen der Formel I gemäß Anspruch 1 , als Inhibitoren der Tyrosinkinase KDR und FLT.

7. Verwendung der Verbindungen der allgemeinen Formel I gemäß Anspruch 1 in Form eines pharmazeutischen Präparats für die enteral, parenterale und orale Applikation.

8. Verwendung der Verbindungen der allgemeinen Formel II 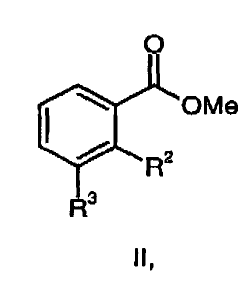 in der
R² und R³ Wasserstoff oder die Gruppe XR¹¹,
X C₂₋₆-Alkyl, C₂₋₆-Alkenyl oder C₂₋₆-Alkinyl
R¹¹ gegebenenfalls mit C₁₋₆-Alkoxy substituiertes Phenyl oder Pyridyl bedeuten, wobei R² und R³ nicht gleichzeitig für Wasserstoff stehen, sowie deren Isomeren und Salze, zur Herstellung eines Arzneimittels zur Behandlung von Tumoren, Psoriasis, Arthritis, wie rheumatoide Arthritis, Hämangioma, Angiofribroma, Augenerkrankungen, wie diabetische Retinopathie, Neovaskulares Glaukom, Nierenerkrankungen, wie Glomerulonephritis, diabetische Nephropatie, maligne Nephrosklerose, thrombische mikroangiopatische Syndrome, Transplantationsabstoßungen und Glomerulopathie, fibrotische Erkrankungen, wie Leberzirrhose, mesangialzellproliferative Erkrankungen, Artheriosklerose und Verletzungen des Nervengewebes.

## Claims

1. Compounds of general formula I 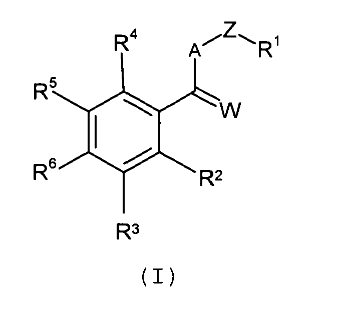 in which
A stands for the group =NR⁷,
W stands for oxygen, or for two hydrogen atoms,
Z stands for a bond, the group =NR¹⁰ or for branched or unbranched C₁₋₁₂-alkyl,
R¹ stands for branched or unbranched C₁₋₆-alkyl; or for C₃₋₁₀-cycloalkyl that is optionally substituted in one or more places with halogen or C₁₋₆-alkyl; or for phenyl, pyridyl, naphthyl, quinolyl, isoquinolyl, indenyl, tetralinyl, indolyl, thienyl, indazolyl, or benzothiazolyl that is unsubstituted or that is optionally substituted in one or more places with halogen, C₁₋₆-alkyl, C₁₋₆-alkoxy or C₁₋₆-alkyl or C₁₋₆-alkoxy that is substituted in one or more places with halogen,
R² and R³ stand for hydrogen, an OH group or the group XR¹¹,
X stands for C₂₋₆-alkyl, C₂₋₆-alkenyl or C₂₋₆-alkinyl,
R¹¹ means phenyl, pyrimidinyl or pyridyl that is unsubstituted or that is optionally substituted in one or more places with halogen, C₁₋₆-alkoxy or hydroxy,
R⁴, R⁵, R⁶ and R⁷ stand for hydrogen,
R⁸ and R¹⁰ stand for hydrogen or C₁₋₆-alkyl, whereby R² and R³ stand for hydrogen, although not simultaneously, and if R² stands for an OH group, R³ does not stand for hydrogen, and if R³ stands for an OH group, R² does not stand for hydrogen, as well as isomers and salts thereof.

2. Use of the compounds of general formula I, according to claim 1, for the production of a pharmaceutical agent for treating tumors, psoriasis; arthritis, such as rheumatoid arthritis, hemangioma, angiofibroma; eye diseases, such as diabetic retinopathy, neovascular glaucoma; renal diseases, such as glomerulonephritis, diabetic nephropathy, malignant nephrosclerosis, thrombic microangiopathic syndrome, transplant rejections and glomerulopathy; fibrotic diseases, such as cirrhosis of the liver, mesangial cell proliferative diseases, arteriosclerosis, and injuries to nerve tissue.

3. Pharmaceutical agents that contain at least one compound according to claim 1.

4. Pharmaceutical agents according to claim 3, for treating tumors, psoriasis; arthritis, such as rheumatoid arthritis, hemangioma, angiofibroma; eye diseases, such as diabetic retinopathy, neovascular glaucoma; renal diseases, such as glomerulonephritis, diabetic nephropathy, malignant nephrosclerosis, thrombic microangiopathic syndrome, transplant rejections and glomerulopathy; fibrotic diseases, such as cirrhosis of the liver, mesangial cell proliferative diseases, arteriosclerosis, and injuries to nerve tissue.

5. Compounds, according to claim 1, with suitable formulations and vehicles.

6. Use of the compounds of formula I according to claim 1 as inhibitors of the tyrosine kinase KDR and FLT.

7. Use of the compounds of general formula I, according to claim 1, in the form of a pharmaceutical preparation for enteral, parenteral and oral administration.

8. Use of the compounds of general formula II 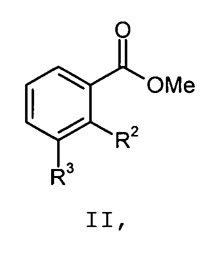 in which
R² and R³ mean hydrogen or the group XR¹¹,
X means C₂₋₆-alkyl, C₂₋₆-alkenyl or C₂₋₆-alkinyl,
R¹¹ means phenyl or pyridyl that is optionally substituted by C₁₋₆-alkoxy, whereby R² and R³ stand for hydrogen, although not simultaneously, as well as isomers and salts thereof, for the production of a pharmaceutical agent for treating tumors, psoriasis; arthritis, such as rheumatoid arthritis, hemangioma, angiofibroma; eye diseases, such as diabetic retinopathy, neovascular glaucoma; renal diseases, such as glomerulonephritis, diabetic nephropathy, malignant nephrosclerosis, thrombic microangiopathic syndrome, transplant rejections and glomerulopathy; fibrotic diseases, such as cirrhosis of the liver, mesangial cell proliferative diseases, arteriosclerosis, and injuries to nerve tissue.

## Revendications

1. Composés de formule générale I 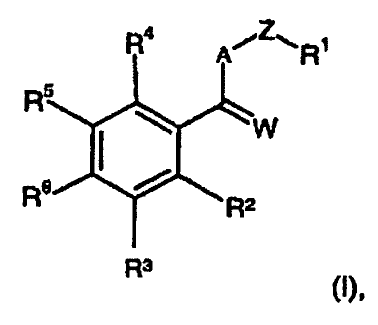 dans laquelle
A représente le groupe =NR⁷,
W représente un atome d'oxygène ou deux atomes d'hydrogène,
Z représente une liaison, le groupe =NR¹⁰ ou un groupe alkyle en C₁-C₁₂ ramifié ou non ramifié,
R¹ représente un groupe alkyle en C₁-C₆ ramifié ou non ramifié, ou un groupe cycloalkyle en C₃-C₁₀ éventuellement une ou plusieurs fois substitué par halogène ou alkyle en C₁-C₆, ou un groupe phényle, pyridyle, naphtyle, quinoléyle, isoquinoléyle, indényle, tétralinyle, indolyle, thiényle, indazolyle ou benzothiazolyle, non substitué ou éventuellement une ou plusieurs fois substitué par halogène, alkyle en C₁-C₆, alcoxy en C₁-C₆, alkyle en C₁-C₆ ou alcoxy en C₁-C₆ une ou plusieurs fois substitués par halogène,
R² et R³ représentent un atome d'hydrogène, un groupe OH ou le groupe XR¹¹,
X représente un groupe alkyle en C₂-C₆, alcényle en C₂-C₆ ou alcynyle en C₂-C₆,
R¹¹ représente un groupe phényle, pyrimidinyle ou pyridyle, non substitué ou éventuellement une ou plusieurs fois substitué par halogène, alcoxy en C₁-C₆ ou hydroxy,
R⁴, R⁵, R⁶ et R⁷ représentent des atomes d'hydrogène,
R⁸ et R¹⁰ représentent un atome d'hydrogène ou un groupe alkyle en C₁-C₆,
R² et R³ ne représentant pas simultanément des atomes d'hydrogène et si R² représente un groupe OH, R³ ne représentant pas un atome d'hydrogène et si R³ représente un groupe OH, R² ne représentant pas un atome d'hydrogène,
ainsi que leurs isomères et sels.

2. Utilisation des composés de formule générale I selon la revendication 1, pour la fabrication d'un médicament destiné au traitement de tumeurs, du psoriasis, de l'arthrite, telle que la polyarthrite rhumatoïde, de l'hémangiome, de l'angiofibrome, de maladies des yeux, telles que la rétinopathie diabétique, le glaucome néovasculaire, de maladies rénales telles que la glomérulonéphrite, la néphropathie diabétique, la néphrosclérose maligne, de syndromes microangiopathiques thrombotiques, de rejets de greffes et de la glomérulopathie, de maladies fibreuses telles que la cirrhose, de maladies prolifératives des cellules mésangiales, de l'artériosclérose et de lésions du tissu nerveux.

3. Médicament, contenant au moins un composé selon la revendication 1.

4. Médicament selon la revendication 3, destiné au traitement de tumeurs, du psoriasis, de l'arthrite, telle que la polyarthrite rhumatoïde, de l'hémangiome ; de l'angiofibrome, de maladies des yeux, telles que la rétinopathie diabétique, le glaucome néovasculaire, de maladies rénales telles que la glomérulonéphrite, la néphropathie diabétique, la néphrosclérose maligne, de syndromes microangiopathiques thrombotiques, de rejets de greffes et de la glomérulopathie, de maladies fibreuses telles que la cirrhose, de maladies prolifératives des cellules mésangiales, de l'artériosclérose et de lésions du tissu nerveux.

5. Composés selon la revendication 1, avec des adjuvants de formulation et véhicules appropriés.

6. Utilisation des composés de formule I selon la revendication 1, en tant qu'inhibiteurs de la tyrosine kinase KDR et FLT.

7. Utilisation des composés de formule I selon la revendication 1, sous forme d'une préparation pharmaceutique pour l'administration entérale, parentérale et orale.

8. Utilisation des composés de formule générale II 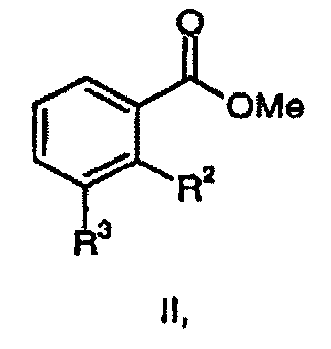 dans laquelle
R² et R³ représentent un atome d'hydrogène ou le groupe XR¹¹,
X représente un groupe alkyle en C₂-C₆, alcényle en C₂-C₆ ou alcynyle en C₂-C₆,
R¹¹ représente un groupe phényle ou pyridyle éventuellement substitué par alcoxy en C₁-C₆,
R² et R³ ne représentant pas simultanément des atomes d'hydrogène, ainsi que de leurs isomères et sels, pour la fabrication d'un médicament destiné au traitement de tumeurs, du psoriasis, de l'arthrite, telle que la polyarthrite rhumatoïde, de l'hémangiome, de l'angiofibrome, de maladies des yeux, telles que la rétinopathie diabétique, le glaucome néovasculaire, de maladies rénales telles que la glomérulonéphrite, la néphropathie diabétique, la néphrosclérose maligne, de syndromes microangiopathiques thrombotiques, de rejets de greffes et de la glomérulopathie, de maladies fibreuses telles que la cirrhose, de maladies prolifératives des cellules mésangiales, de l'artériosclérose et de lésions du tissu nerveux.
